(19)

**Europäisches Patentamt**
**European Patent Office**
**Office européen des brevets**

(11)  **EP 2 562 251 B1**

(12)  **EUROPEAN PATENT SPECIFICATION**

(45) Date of publication and mention
of the grant of the patent:
**17.08.2016 Bulletin 2016/33**

(51) Int Cl.:
*C12N 9/04* (2006.01)          *C12Q 1/00* (2006.01)
*C12Q 1/26* (2006.01)          *C12Q 1/54* (2006.01)

(21) Application number: **11006940.8**

(22) Date of filing: **25.08.2011**

(54) **Cholesterol oxidase**

Cholesterinoxidase

Oxydase de cholestérol

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**27.02.2013 Bulletin 2013/09**

(73) Proprietors:
• **Roche Diagnostics GmbH**
**68305 Mannheim (DE)**
Designated Contracting States:
**DE**
• **F.Hoffmann-La Roche AG**
**4070 Basel (CH)**
Designated Contracting States:
**AL AT BE BG CH CY CZ DK EE ES FI FR GB GR
HR HU IE IS IT LI LT LU LV MC MK MT NL NO PL
PT RO RS SE SI SK SM TR**
• **Ultizyme International Ltd.**
**Meguro-ku,
Tokyo 152-0013 (JP)**

(72) Inventors:
• **Kojima, Katsuhiro**
**Tokyo 152-0013 (JP)**
• **Mori, Kazushige**
**Tokyo 152-0013 (JP)**
• **Sode, Koji**
**Tokyo 183-8538 (JP)**

(74) Representative: **Silber, Anton et al
Roche Diagnostics GmbH
Patentabteilung
68298 Mannheim (DE)**

(56) References cited:
• VRIELINK ALICE ET AL: "Cholesterol oxidase: biochemistry and structural features", FEBS JOURNAL, vol. 276, no. 23, December 2009 (2009-12), pages 6826-6843, XP002669677, ISSN: 1742-464X, DOI: 10.1111/J.1742-4658.2009.07377.X
• POLLEGIONI LOREDANO ET AL: "Cholesterol oxidase: biotechnological applications", FEBS JOURNAL, vol. 276, no. 23, December 2009 (2009-12), pages 6857-6870, XP002669678, ISSN: 1742-464X, DOI: 10.1111/J.1742-4658.2009.07379.X
• YUE Q KIMBERLEY ET AL: "Crystal structure determination of cholesterol oxidase from Streptomyces and structural characterization of key active site mutants", BIOCHEMISTRY, vol. 38, no. 14, 6 April 1999 (1999-04-06) , pages 4277-4286, XP002669679, ISSN: 0006-2960
• TOYAMA MITSUTOSHI ET AL: "Alteration of substrate specificity of cholesterol oxidase from Streptomyces sp. by site-directed mutagenesis", PROTEIN ENGINEERING, vol. 15, no. 6, June 2002 (2002-06), pages 477-483, XP002669680, ISSN: 0269-2139
• YAN SUN ET AL: "Improvement of the thermostability and enzymatic activity of cholesterol oxidase by site-directed mutagenesis", BIOTECHNOLOGY LETTERS, vol. 33, no. 10, 24 June 2011 (2011-06-24) , pages 2049-2055, XP019952703, SPRINGER NETHERLANDS, DORDRECHT ISSN: 1573-6776, DOI: 10.1007/S10529-011-0669-6

**(Cont. next page)**

- DATABASE UniProt [Online] 23 November 2004 (2004-11-23), "SubName: Full=Putative cholesterol oxidase;", XP002683266, retrieved from EBI accession no. UNIPROT:Q5Z338 Database accession no. Q5Z338 & ISHIKAWA J ET AL: "The complete genomic sequence of Nocardia farcinica IFM 10152", PROCEEDINGS OF THE NATIONAL ACADEMY OF SCIENCES, NATIONAL ACADEMY OF SCIENCES, US, vol. 101, no. 41, 12 October 2004 (2004-10-12), pages 14925-14930, XP002995067, ISSN: 0027-8424, DOI: 10.1073/PNAS.0406410101
- DATABASE UniProt [Online] 29 May 2007 (2007-05-29), "SubName: Full=Cholesterol oxidase; EC=1.1.3.6; Flags: Precursor;", XP002683267, retrieved from EBI accession no. UNIPROT:A4X855 Database accession no. A4X855
- DATABASE UniProt [Online] 2 November 2010 (2010-11-02), "SubName: Full=Putative cholesterol oxidase;", XP002683268, retrieved from EBI accession no. UNIPROT:E0TBM8 Database accession no. E0TBM8
- DOUKYU NORIYUKI: "Characteristics and biotechnological applications of microbial cholesterol oxidases", APPLIED MICROBIOLOGY AND BIOTECHNOLOGY, vol. 83, no. 5, July 2009 (2009-07), pages 825-837, XP002669681, ISSN: 0175-7598, DOI: 10.1007/S00253-009-2059-8
- PARRA A ET AL: "Bioanalytical device based on cholesterol oxidase-bonded SAM-modified electrodes", ANALYTICAL AND BIOANALYTICAL CHEMISTRY, vol. 388, no. 5-6, 6 March 2007 (2007-03-06), pages 1059-1067, XP019537456, SPRINGER, BERLIN, DE ISSN: 1618-2650, DOI: 10.1007/S00216-007-1187-1
- DATABASE UNIPROT [Online] 05 October 2010 'SubName: Full=Cholesterol oxidase (CHOD);' Retrieved from EBI, accession no. UNIPROT:D9WHR8 Database accession no. D9WHR8

**Description**

Technical Field

The present invention relates to a cholesterol oxidase for use in a kit and a sensor for the measurement of cholesterol. More particularly, the present invention relates to a mutant cholesterol oxidase having reduced oxidase activity.

Background Art

The concentration of lipoproteins in blood is quite important in clinical tests. Lipoproteins in blood can be divided in High Density Lipoproteins and Low Density Lipoproteins, both having different biological functions. As a measure of the lipoprotein content in blood, cholesterol associated with the lipoproteins is measured. In biological samples like blood, cholesterol is present in the lipoproteins in the form of cholesterol esters. To measure the lipoprotein associated cholesterol levels the cholesterol esters are split by the use of enzymes like cholesterol esterase and the freed cholesterol is then determined. The cholesterol concentration in blood may be measured using an enzyme having specificity to cholesterol.

Cholesterol oxidases have been isolated from various kinds of strains and it has been suggested that cholesterol may be analyzed using such enzymes. For example, Vrielink et al., FEBS J (2009), 276 (23):6826-6848 provides an overview of structure and properties of wild type and mutant cholesterol oxidases from a variety of organisms. Particular mutant cholesterol oxidases from different organisms are disclosed to show altered substrate specificity (Pollegioni et al., FEBS J. (2009), 276(23):6857-70; Toyama et al., Protein Engineering (2002), 15(6):477-483), improved thermostability (Pollegioni, *supra*, Yan Sun et a., Biotechnology Letters (2011), 33(10):2049-2055) and reduced activity for either oxidation or isomerization (Vrielink, *supra*, Yue et al., Biochemistry (1999), 38(14):4277-4286, Toyama *supra*). However, none of those documents disclose a mutant cholesterol oxidase consisting of an amino acid sequence set forth in SEQ ID NO: 1 and having an oxidase activity of less than its dehydrogenase activity, wherein the amino acid residue at position 159 is an amino acid residue selected from Phe, Leu, Val, Cys, Ile, Ala, Gln, Tyr, Lys, and Ser. Cholesterol oxidase is a FAD-dependent enzyme which catalyzes a reaction where cholesterol is oxidized to generate cholest-4-en-3-one, with generating the reduced form of FAD (FADH2). FADH2 in turn transmits electron to an electron acceptor and is converted to its oxidized form. In the presence of oxygen, FADH2 preferentially transmits electrons to the oxygen molecule rather than to artificial electron acceptors (also referred to as mediators or electron mediators). Thus, when cholesterol is assayed by cholesterol oxidase with mediators, the assay results will be greatly affected by the dissolved oxygen level in the reaction system. Such a disadvantage will be particularly noted in clinical tests of blood samples by a point-of-care testing device utilizing an artificial electron acceptor. The enzyme used for the enzyme sensor strips employing artificial electron mediators desirably have low activity toward oxygen.

Accordingly, the object of the present invention is to provide an enzyme, in particular a cholesterol oxidase, whose activity is less affected by the dissolved oxygen level.

Disclosure of the Invention

The present application provides an enzyme, in particular a cholesterol oxidase, whose activity is less affected by the dissolved oxygen level. More specifically, this has been achieved by reducing the oxidase activity of an enzyme that in its wild-type form predominantly shows an oxidase activity and by preferably at the same time increasing the enzyme's dehydrogenase activity. As will be described in more detail below, this has been achieved by mutating the wild type enzyme.

The present inventors have prepared various mutants of a cholesterol oxidase and surprisingly found that a certain type of mutants exhibit reduced oxidase activity while substantially retaining dehydrogenase activity, in particular dye-mediated dehydrogenase activity.

The present invention provides a mutant cholesterol oxidase as defined in independent claim 1. Preferred embodiments of the mutant cholesterol oxidase are the subject matter of the claims depending from claim 1.

In a preferred embodiment, the mutant cholesterol oxidase of the invention has a reduced oxidase activity as compared to the wild-type cholesterol oxidase, and preferably has an increased dehydrogenase activity compared to the wild-type cholesterol oxidase. Preferably, the mutant cholesterol oxidase of the invention has an oxidase activity of 30% or less of that of the wild-type cholesterol oxidase, and preferably also has a dehydrogenase activity of 50% or more of the wild-type cholesterol oxidase. Also preferably, the mutant cholesterol oxidase of the invention has an increased dehydrogenase activity compared to the wild-type cholesterol oxidase.

In another aspect, the present invention provides an isolated polynucleotide encoding the mutant cholesterol oxidase of the present invention.

In yet another aspect, the present invention provides a vector comprising the polynucleotide of the invention.

In still another aspect, the present invention provides a host cell transformed with a vector of the invention.

In another aspect, the present invention provides a method for assaying cholesterol in a sample, comprising contacting the sample with the cholesterol oxidase of the invention and measuring the amount of the cholesterol oxidized by cholesterol oxidase.

In another aspect, the present invention provides a device for assaying cholesterol in a sample comprising the cholesterol oxidase of the invention and preferably an electron mediator.

In yet another aspect, the present invention provides a kit for assaying cholesterol in a sample comprising the cholesterol oxidase of the invention and preferably an electron mediator.

In another aspect, the present invention provides an enzyme electrode having the cholesterol oxidase of the invention which is immobilized on the electrode.

In another aspect, the present invention provides an enzyme sensor for assaying cholesterol comprising the enzyme electrode of the invention as a working electrode.

Detailed Description of the Invention

The term "mutant" of a protein as used herein refers to a variant protein containing substitution in one or more of the amino acid residues on the protein at the indicated position(s). The term mutant is also used for a polynucleotide encoding such a mutant protein.

The phrase "a position corresponding to" as used herein means the position of an amino acid residue in a query amino acid sequence that is aligned with the amino acid residue in a reference amino acid sequence using a software AlignX of Vector NTI with default parameters (available from Invitrogen; see, Lu, G., and Moriyama, E. N. (2004) Vector NTI, a balanced all-in-one sequence analysis suite. Brief Bioinform 5, 378-88). Thus, "amino acid (AA) residue at a position corresponding to the position Y of the amino acid sequence set forth in SEQ ID NO: X" means the AA residue in a query amino acid sequence that is aligned with AA Y of SEQ ID NO: X when the query amino acid sequence is aligned with SEQ ID NO: X using AlignX of Vector NTI with default parameters. It should be noted that the AA Y of SEQ ID NO: X itself is also encompassed by this term.

The mutant cholesterol oxidase of the invention exhibit decreased oxidase (or Ox) activity while substantially retaining dehydrogenase (or Dh) activity.

As used herein "oxidase activity" is an enzymatic activity of the cholesterol oxidase to catalyze oxidation of cholesterol to generate cholest-4-en-3-one with utilizing oxygen as an electron acceptor. The oxidase activity may be assayed by measuring the amount of generated $H_2O_2$ by any methods known in the art, for example, by reagents for $H_2O_2$ detection such as 4AA/TODB/POD (4-aminoantipyrine/N,N-Bis(4-sulfobutyl)-3-methylaniline disodium salt/horseradish peroxidase)or by Pt electrode. As used herein in the context of the relative or quantitative activity, the oxidase activity is specifically defined to be the mole amount of the substrate (cholesterol) oxidized per unit time measured by the amount of generated $H_2O_2$ at 25 °C in 10 mM PPB, pH 7.0, 1.5 mM TODB, 2 U/ml horseradish peroxidase (POD), and 1.5 mM 4-aminoanti-pyrine (4AA). The formation of quinoneimine dye may be measured spectrophotometrically at 546 nm.

As used herein, "dehydrogenase activity" is an enzymatic activity of the cholesterol oxidase to catalyze oxidation of cholesterol to generate cholest-4-en-3-one with utilizing an electron mediator other than oxygen as an electron acceptor. The dehydrogenase activity may be assayed by measuring the amount of electron transferred to the mediator using, for example, mPMS/DCIP (1-methoxy-5-methylphenazinium methylsulfate/ 2,6-dichloroindophenol), cPES (trifluoro-acetate-1-(3-carboxy-propoxy)-5-thyl-phenanzinium, NA BM31_1144 (N,N-bis-(hydroxyethyl)¬3-methoxy-nitrosoaniline hydrochloride, NA BM31_1008 (N,N-bis-hydroxy¬ethyl-4-nitrosoaniline) and N-N-4-dimethyl-nitrosoaniline.

As used herein in the context of the relative or quantitative activity, the dehydrogenase activity is specifically defined to be the mole amount of the substrate (cholesterol) oxidized per unit time measured by the amount of electron transferred to the mediator at 25 °C in 10 mM PPB (pH 7.0), 0.6 mM DCIP, and 6 mM methoxy PMS (mPMS).

The mutant cholesterol oxidase of the invention has a reduced oxidase activity as compared to the wild-type cholesterol oxidase, while substantially retaining the dehydrogenase activity.

Preferably, the mutant cholesterol oxidase of the invention has an oxidase activity of 50% or less of that of the wild-type cholesterol oxidase. More preferably, the mutant cholesterol oxidase of the invention has an oxidase activity of 40% or less, more preferably 30% or less, even more preferably 20% or less, most preferably 15% or less of that of the wild-type cholesterol oxidase. Also preferably, the mutant cholesterol oxidase of the invention has a dehydrogenase activity of 50% or more of the wild-type cholesterol oxidase. More preferably, the mutant cholesterol oxidase of the invention has a dehydrogenase activity of 70% or more, more preferably 90% or more, even more preferably 100% or more, most preferably more than 100% of the wild-type cholesterol oxidase.

In the wild-type cholesterol oxidase, the oxidase activity is about 300 times higher than the dehydrogenase activity. When dissolved oxygen is present in the assay system, the electron generated by the oxidation of the substrate will be preferentially transferred to the oxygen. Thus the enzyme activity measured in the presence of electron mediator will be greatly affected by the dissolved oxygen concentration. In contrast, the mutant cholesterol oxidase of the invention has the ratio of dehydrogenase/oxidase activity of about 2.0 or more, preferably 4.0 or more, more preferably 10 or more. Since the dehydrogenase activity exceed the oxidase activity, the enzyme activity of the cholesterol oxidase of the present invention will be less affected by the dissolved oxygen concentration, which is advantageous in utilizing the cholesterol oxidase in clinical diagnosis with a blood sample.

It should be understood that the numbering of the amino acid sequence in this application begins at the initial Met and that the claimed mutant cholesterol oxidase may or may not have the signal peptide.

In another aspect, the present invention provides an isolated polynucleotide encoding the mutant cholesterol oxidase of the present invention. The nucleotide sequence of polynucleotides coding for cholesterol oxidase may be easily obtained from public databases. The polynucleotide encoding the wild type cholesterol oxidase may be cloned from the genome of respective organisms using PCR or other known techniques. Mutations may be introduced by site-directed mutagenesis, PCR mutagenesis or any other techniques well known in the art. The amino acid residue to be mutated may be identified using any of software for sequence alignment available in the art. Alternatively, polynucleotide coding for the mutant cholesterol oxidase may be prepared by PCR using a series of chemically synthesized oligonucleotides, or fully synthesized.

The mutated cholesterol oxidase may be prepared by inserting the mutant gene into an appropriate expression vector and introducing the vector into an appropriate host cell, such as E. coli cells. The transformant is cultured and the cholesterol oxidase expressed in the transformant may be collected from the cells or culture medium.

The recombinant cholesterol oxidase thus obtained may be purified by any of the purification techniques known in the art, including ion exchange column chromatography, affinity chromatography, liquid chromatography, filtration, ultrafiltration, salt precipitation, solvent precipitation, immunoprecipitation, gel electrophoresis, isoelectric electrophoresis and dialysis.

Thus, the present invention also encompasses a vector comprising the polynucleotide encoding the mutant cholesterol oxidase, a host cell transformed with such a vector, and a method for preparing the mutant cholesterol oxidase of the invention by culturing the transformant, collecting and purifying the mutant cholesterol oxidase from the culture.

The invention also encompasses a method for assaying cholesterol, HDL cholesterol or LDL cholesterol in a sample. The method comprises contacting the sample with the cholesterol oxidase of the invention and measuring the amount of the cholesterol oxidized by the cholesterol oxidase.

In another aspect, the present invention provides a device for assaying cholesterol, HDL cholesterol or LDL cholesterol in a sample comprising the cholesterol oxidase of the invention and an electron mediator.

The assay device may have a similar structure as any of conventional, commercially available amperometric biosensor test strips for monitoring the blood cholesterol level. One example of such a device has two electrodes (working electrode and reference or counter electrode) positioned on an insulating substrate, a reagent port and a sample receiver. The reagent port contains the mutated cholesterol oxidase of the invention and a mediator. When a sample such as blood sample is added to the sample receiver, cholesterol contained in the sample will react with cholesterol oxidase to generate current, which is indicative of the amount of cholesterol in the sample. Typical examples of electrochemical sensors suited for the determination of enzyme substrates are known, e.g. from WO 2004/113900 and US 5,997,817. As an alternative to electrochemical sensors, optical detection technologies might be used. Typically, such optical devices are based on color changes that occur in a reagent system comprising the enzyme, an electron mediator and an indicator. The color changes can be quantified using fluorescence, absorption or remission measurements. Typical examples of optical devices suited for the determination of enzyme substrates are known, e.g. from US 7,008,799, US 6,036,919, and US 5,334,508.

In yet another aspect, the present invention provides a kit for assaying cholesterol, HDL cholesterol or LDL cholesterol in a sample comprising the cholesterol oxidase of the invention and an electron mediator.

A kit for the measurement of cholesterol, HDL cholesterol or LDL cholesterol may be constructed using the enzyme of the present invention. In addition to the cholesterol oxidase of the invention, the kit contains buffer necessary for the measurement, appropriate mediator and, if necessary, further enzymes such as cholesterol esterase, a standard solution of cholesterol for the preparation of a calibration curve and an instruction for use. The cholesterol oxidase of the present invention may be provided in various forms, for example, as a freeze-dried reagent or as a solution in an appropriate storage solution.

In another aspect, the present invention provides an enzyme electrode having the cholesterol oxidase of the invention which is immobilized on the electrode.

In another aspect, the present invention provides an enzyme sensor for assaying cholesterol comprising the enzyme electrode of the invention as a working electrode.

The concentration of the cholesterol in a sample may be determined by measuring the amount of electron generated by the enzyme reaction. Various sensor systems have been known in the art, including carbon electrode, metal electrode, and platinum electrode. The mutated cholesterol oxidase of the present invention is immobilized on the electrodes. Examples of the means for immobilization include cross-linking, encapsulating into a macromolecular matrix, coating with a dialysis membrane, optical cross-linking polymer, electroconductive polymer, oxidation-reduction polymer, and any combination thereof.

When measurement is conducted in an amperometric system using carbon electrode, gold electrode or platinum electrode provided with an immobilized enzyme is used as a working electrode, together with a counter electrode (such as platinum electrode) and a reference electrode (such as Ag/AgCl electrode). The electrodes are inserted into a buffer containing a mediator and kept at predetermined temperature. Predetermined voltage is applied to the working electrode, then a sample is added and increased value in electric current is measured. Examples of the mediator used in the assay include potassium ferricyanide, ferrocene, osmium derivative, ruthenium derivative, phenazine methosulfate, etc. It is generally also possible to use so-called two-electrode systems with one working electrode and one counter or pseudo-reference electrode.

Further, cholesterol may be assayed using an immobilized electron mediator in an amperometric system using carbon electrode, gold electrode, or platinum electrode. The enzyme is immobilized on the electrode together with an electron mediator such as potassium ferricyanide, ferrocene, osmium derivative, phenazine methosulfate in a macromolecular matrix by means of adsorption or covalent bond to prepare a working electrode. It is inserted into buffer together with a counter electrode (such as platinum electrode) and a reference electrode (such as Ag/AgCl electrode), and kept at a predetermined temperature. Predetermined voltage is applied to the working electrode, then the sample is added and increased value in electric current is measured.

It is to be understood that whenever this application refers to cholesterol as an analyte also other analytes that can be converted to cholesterol, like e.g. HDL cholesterol or LDL cholesterol, shall be encompassed. A person of skill in the art knows that a cholesterol esterase enzyme may be needed to set free cholesterol from cholesterol esters which naturally occur in sample materials like blood or blood fractions.

Examples

The present invention will be illustrated in detail by way of the Examples below, although the present invention shall not be limited to those Examples.

**Example 1: Plasmid expressing ChOx of *Streptomyces* sp. strain SA-COO**

pET28 ChOx_Nhis was used as a plasmid expressing ChOx of *Streptomyces* sp. strain SA-COO. This plasmid has the DNA fragment of the ChOx structural gene derived from *Streptomyces* sp. strain SA-COO which is inserted in the *Nhe*I/*Hind*III cloning site of a vector pET28a. The ChOx gene in this plasmid is controlled by a T7 promoter. The pET28 ChOx_Nhis contains a kanamycin resistance gene.

**Example 2: Mutagenesis of ChOx structural gene derived from *Streptomyces* sp. strain SA-COO**

(1) Mutagenesis of residues 159, 228, and 396

The *Streptomyces* sp. strain SA-COO-derived ChOx structural gene contained in the pET28 ChOx_Nhis obtained in Example 1 was mutagenized such that methionine at residue 159, valine at residue 228, and phenylalanine at residue 396 in ChOx encoded by this gene were substituted by other amino acid residues.

Specifically, codon (ATG) for methionine at residue 159, codon (GTT) for valine at residue 228, and codon (TTT) for phenylalanine at residue 396 in the ChOx structural gene contained in the plasmid pET28 ChOx_Nhis described in Example 1 were substituted by other amino acid codons using a commercially available site-directed mutagenesis kit (Stratagene Corp., QuikChange II Site-Directed Mutagenesis Kit).

The sequences of forward and reverse primers used in the amino acid residue substitution are shown in the tables below.

In notation representing a mutation, the number represents a position in the amino acid sequence containing the signal sequence of ChOx; the alphabet described before the number represents an amino acid residue before amino acid substitution; and the alphabet described after the number represents an amino acid residue after amino acid substitution. For example, M159A represents the substitution of methionine at residue 159 to alanine.

In PCR reaction, a reaction solution of the composition shown below was subjected to reaction at 95°C for 30 seconds and then 15 repetitive cycles each involving 95°C for 30 seconds, 55°C for 1 minute, and 68°C for 8 minutes, followed by 68°C for 30 minutes and then kept at 4°C.

***Composition of reaction solution***

| | |
|---|---|
| Template DNA (5 ng/μL) | 2 μL |
| 10×reaction buffer | 5 μL |
| Forward primer (100 ng/μL) | 1.25 μL |
| Reverse primer (100 ng/μL) | 1.25 μL |
| dNTP | 1 μL |
| Distilled water | 38.5 μL |
| DNA polymerase | 1 μL |
| Total | 50 μL |

After the PCR reaction, 0.5 μL of *Dpn*I was added to the reaction solution and incubated at 37°C for 1 hour to degrade the template plasmid.

*Escherichia coli* DH5α (supE44, ΔlacU169 (φ80lacZΔM15), hsdR17, recA1, endA1, gyrA96, thi-1, relAI) competent cells were transformed with the obtained reaction solution. From colonies grown on an LB agar medium (1% Bacto tryptone, 0.5% yeast extracts, 1% sodium chloride, 1.5% agar) containing kanamycin (50 μg/mL), plasmid DNA was prepared and sequenced to confirm that the mutation of interest was introduced in the ChOx structural gene.

The plasmid confirmed to have the introduced mutation was digested with restriction enzymes *Nhe*I and *Hind*III to excise the mutagenized ChOx structural gene, which was in turn inserted to a pET28a vector. DH5α was transformed with this plasmid, and a plasmid was extracted from the obtained colonies to obtain a ChOx mutant expression plasmid.

**Table 1:** Forward primer for M159

| Amino acid substitution | Primer name | Sequence | SEQ ID NO. |
|---|---|---|---|
| M159A | M159AFw | 5'GGTTAACGGTGGC<u>GC</u>GGCGGTGGAACCG 3' | 49 |
| M159C | M159CFw | 5'GGTTAACGGTGGC<u>TGC</u>GCGGTGGAACCG 3' | 50 |
| M159D | M159DFw | 5'GGTTAACGGTGGC<u>GAC</u>GCGGTGGAACCG 3' | 51 |
| M159E | M159EFw | 5'GGTTAACGGTGGC<u>GAA</u>GCGGTGGAACCG 3' | 52 |
| M159F | M159FFw | 5'GGTTAACGGTGGC<u>TT</u>CGCGGTGGAACCG 3' | 53 |
| M159G | M159GFw | 5'GGTTAACGGTGGCGGTGC<u>GG</u>TGGAACCG 3' | 54 |
| M159H | M159HFw | 5'GGTTAACGGTGGC<u>CAC</u>GCGGTGGAACCG 3' | 55 |
| M159I | M159IFw | 5'GGTTAACGGTGGC<u>ATC</u>GCGGTGGAACCG 3' | 56 |
| M159K | M159KFw | 5'GGTTAACGGTGGC<u>AAA</u>GCGGTGGAACCG 3' | 57 |
| M159L | M159LFw | 5'GGTTAACGGTGGC<u>CT</u>GGCGGTGGAACCG 3' | 58 |
| M159N | M159NFw | 5'GGTTAACGGTGGC<u>AAC</u>GCGGTGGAACCG 3' | 59 |
| M159P | M159PFw | 5'GGTTAACGGTGGC<u>CC</u>GGCGGTGGAACCG 3' | 60 |
| M159Q | M159QFw | 5'GGTTAACGGTGGC<u>CAG</u>GCGGTGGAACCG 3' | 61 |
| M159R | M159RFw | 5'GGTTAACGGTGGC<u>TT</u>CGCGGTGGAACCG 3' | 62 |
| M159S | M159SFw | 5'GGTTAACGGTGGC<u>TCT</u>GCGGTGGAACCG 3' | 63 |
| M159T | M159TFw | 5'GGTTAACGGTGGC<u>ACC</u>GCGGTGGAACCG 3' | 64 |
| M159V | M159VFw | 5'GGTTAACGGTGGC<u>GTT</u>GCGGTGGAACCG 3' | 65 |
| M159W | M159WFw | 5'GGTTAACGGTGGC<u>GGC</u>GCGGTGGAACCG 3' | 66 |
| M159Y | M159YFw | 5'GGTTAACGGTGGC<u>TAC</u>GCGGTGGAACCG 3' | 67 |

**Table 2:** Reverse primer for M159

| Amino acid substitution | Primer name | Sequence | SEQ ID NO. |
|---|---|---|---|
| M159A | M159ARv | 5'CGGTTCCACCGC<u>CGC</u>GCCACCGTTAACC 3' | 68 |
| M159C | M159CRv | 5'CGGTTCCACCGC<u>GCA</u>GCCACCGTTAACC 3' | 69 |
| M159D | M159DRv | 5'CGGTTCCACCGC<u>GTC</u>GCCACCGTTAACC 3' | 70 |
| M159E | M159ERv | 5'CGGTTCCACCGC<u>TTC</u>GCCACCGTTAACC 3' | 71 |
| M159F | M159FRv | 5'CGGTTCCACCGC<u>GAA</u>GCCACCGTTAACC 3' | 72 |
| M159G | M159GRv | 5'CGGTTCCACCGC<u>ACC</u>GCCACCGTTAACC 3' | 73 |
| M159H | M159HRv | 5'CGGTTCCACCGC<u>GTG</u>GCCACCGTTAACC 3' | 74 |

(continued)

| Amino acid substitution | Primer name | Sequence | SEQ ID NO. |
|---|---|---|---|
| M159I | M159IRv | 5'CGGTTCCACCGC<u>GAT</u>GCCACCGTTAACC 3' | 75 |
| M159K | M159KRv | 5'CGGTTCCACCGC<u>TTT</u>GCCACCGTTAACC 3' | 76 |
| M159L | M159LRv | 5'CGGTTCCACCGCC<u>AG</u>GCCACCGTTAACC 3' | 77 |
| M159N | M159NRv | 5'CGGTTCCACCGC<u>GTT</u>GCCACCGTTAACC 3' | 78 |
| M159P | M159PRv | 5'CGGTTCCACCGCC<u>GG</u>GCCACCGTTAACC 3' | 79 |
| M159Q | M159QRv | 5'CGGTTCCACCGC<u>CTG</u>GCCACCGTTAACC 3' | 80 |
| M159R | M159RRv | 5'CGGTTCCACCGC<u>GAA</u>GCCACCGTTAACC 3' | 81 |
| M159S | M159SRv | 5'CGGTTCCACCGC<u>AGA</u>GCCACCGTTAACC 3' | 82 |
| M159T | M159TRv | 5'CGGTTCCACCGC<u>GGT</u>GCCACCGTTAACC 3' | 83 |
| M159V | M159VRv | 5'CGGTTCCACCGC<u>AAC</u>GCCACCGTTAACC 3' | 84 |
| M159W | M159WRv | 5'CGGTTCCACCGC<u>GCC</u>GCCACCGTTAACC 3' | 85 |
| M159Y | M159YRv | 5'CGGTTCCACCGC<u>GTA</u>GCCACCGTTAACC 3' | 86 |

**Table 3:** Forward primer for V228

| Amino acid substitution | Primer name | Sequence | SEQ ID NO. |
|---|---|---|---|
| V228A | V228AFw | 5'GGGTACCGTGTTT<u>GCG</u>CCGAACGTGTATG 3' | 87 |
| V228C | V228CFw | 5'GGGTACCGTGTTT<u>TGC</u>CCGAACGTGTATG 3' | 88 |
| V228D | V228DFw | 5'GGGTACCGTGTTT<u>GAC</u>CCGAACGTGTATG 3' | 89 |
| V228E | V228EFw | 5'GGGTACCGTGTTT<u>GAA</u>CCGAACGTGTATG 3' | 90 |
| V228F | V228FFw | 5'GGGTACCGTGTTT<u>TTC</u>CCGAACGTGTATG 3' | 91 |
| V228G | V228GFw | 5'GGGTACCGTGTTT<u>GGT</u>CCGAACGTGTATG 3' | 92 |
| V228H | V228HFw | 5'GGGTACCGTGTTT<u>CAC</u>CCGAACGTGTATG 3' | 93 |
| V228I | V228IFw | 5'GGGTACCGTGTTT<u>ATC</u>CCGAACGTGTATG 3' | 94 |
| v228K | V228KFw | 5'GGGTACCGTGTTT<u>AAA</u>CCGAACGTGTATG 3' | 95 |
| V228L | V228LFw | 5'GGGTACCGTGTTT<u>CTG</u>CCGAACGTGTATG 3' | 96 |
| V228M | V228MFw | 5'GGGTACCGTGTTT<u>ATG</u>CCGAACGTGTATG 3' | 97 |
| V228N | V228NFw | 5'GGGTACCGTGTTT<u>AAC</u>CCGAACGTGTATG 3' | 98 |
| V228P | V228PFw | 5'GGGTACCGTGTTT<u>CCG</u>CCGAACGTGTATG 3' | 99 |
| V228Q | V228QFw | 5'GGGTACCGTGTTT<u>CAG</u>CCGAACGTGTATG 3' | 100 |
| V228R | V228RFw | 5'GGGTACCGTGTTT<u>TTC</u>CCGAACGTGTATG 3' | 101 |
| V228S | V228SFw | 5'GGGTACCGTGTTT<u>TCT</u>CCGAACGTGTATG 3' | 102 |
| V228T | V228TFw | 5'GGGTACCGTGTTT<u>ACC</u>CCGAACGTGTATG 3' | 103 |
| V228W | V228WFw | 5'GGGTACCGTGTTT<u>GGC</u>CCGAACGTGTATG 3' | 104 |
| V228Y | V228YFw | 5'GGGTACCGTGTTT<u>TAC</u>CCGAACGTGTATG 3' | 105 |

**Table 4:** Reverse primer for V228

| Amino acid substitution | Primer name | Sequence | SEQ ID NO. |
|---|---|---|---|
| V228A | V228ARv | 5'CATACACGTTCGG<u>CGC</u>AAACACGGTACCC 3' | 106 |
| V228C | v228CRv | 5'CATACACGTTCGG<u>GCA</u>AAACACGGTACCC 3' | 107 |
| V228D | V228DRv | 5'CATACACGTTCGG<u>GTC</u>AAACACGGTACCC 3' | 108 |
| V228E | V228ERv | 5'CATACACGTTCGG<u>TTC</u>AAACACGGTACCC 3' | 109 |
| V228F | V228FRv | 5'CATACACGTTCGG<u>ACC</u>AAACACGGTACCC 3' | 110 |
| V228G | V228GRv | 5'CATACACGTTCGG<u>ACC</u>AAACACGGTACCC 3' | 111 |
| V228H | V228HRv | 5'CATACACGTTCGG<u>GTG</u>AAACACGGTACCC 3' | 112 |
| V228I | V228IRv | 5'CATACACGTTCGG<u>GAT</u>AAACACGGTACCC 3' | 113 |
| V228K | V228KRv | 5'CATACACGTTCGG<u>TTT</u>AAACACGGTACCC 3' | 114 |
| V228L | V228LRv | 5'CATACACGTTCGG<u>CAG</u>AAACACGGTACCC 3' | 115 |
| V228M | V228MRv | 5'CATACACGTTCGG<u>CAT</u>AAACACGGTACCC 3' | 116 |

(continued)

| Amino acid substitution | Primer name | Sequence | SEQ ID NO. |
|---|---|---|---|
| V228N | V228NRv | 5'CATACACGTTCGGGTTAAACACGGTACCC 3' | 117 |
| V228P | V228PRv | 5'CATACACGTTCGGCGGAAACACGGTACCC 3' | 118 |
| V228Q | V228QRv | 5'CATACACGTTCGGCTGAAACACGGTACCC 3' | 119 |
| V228R | V228RRv | 5'CATACACGTTCGGGAAAAACACGGTACCC 3' | 120 |
| V228S | V228SRv | 5'CATACACGTTCGGAGAAAACACGGTACCC 3' | 121 |
| V228T | V228TRv | 5'CATACACGTTCGGGGTAAACACGGTACCC 3' | 122 |
| V228W | V228WRv | 5'CATACACGTTCGGGCCAAACACGGTACCC 3' | 123 |
| V228Y | V228YRv | 5'CATACACGTTCGGGTAAAACACGGTACCC 3' | 124 |

**Table 5: Forward primer for F396**

| Amino acid substitution | Primer name | Sequence | SEQ ID NO. |
|---|---|---|---|
| F396A | F396AFw | 5'GATAGCTCTGTTGCGGCCGAAATTGCACC 3' | 125 |
| F396C | F396CFw | 5'GATAGCTCTGTTTGCGCCGAAATTGCACC 3' | 126 |
| F396D | F396DEw | 5'GATAGCTCTGTTGACGCCGAAATTGCACC 3' | 127 |
| F396E | F396EFw | 5'GATAGCTCTGTTGAAGCCGAAATTGCACG 3' | 128 |
| F396G | F396GFw | 5'GATAGCTCTGTTGGTGCCGAAATTGCACC 3' | 129 |
| F396H | F396HFw | 5'GATAGCTCTGTTCACGCCGAAATTGCACC 3' | 130 |
| F396I | F396IFw | 5'GATAGCTCTGTTATCGCCGAAATTGCACC 3' | 131 |
| F396K | F396KFw | 5'GATAGCTCTGTTAAAGCCGAAATTGCACC 3' | 132 |
| F396L | F396LFw | 5'GATAGCTCTGTTCTGGCCGAAATTGCACC 3' | 133 |
| F396M | F396MFw | 5'GATAGCTCTGTTATGGCCGAAATTGCACC 3' | 134 |
| F396N | F396NFw | 5'GATAGCTCTGTTAACGCCGAAATTGCACC 3' | 135 |
| F396P | F396PFw | 5'GATAGCTCTGTTCCGGCCGAAATTGCACC 3' | 136 |
| F396Q | F396QFw | 5'GATAGCTCTGTTCAGGCCGAAATTGCACC 3' | 137 |
| F396R | F396RFw | 5'GATAGCTCTGTTTTCGCCGAAATTGCACC 3' | 138 |
| F396S | F396SFw | 5'GATAGCTCTGTTTCTGCCGAAATTGCACC 3' | 139 |
| F396T | F396TFw | 5'GATAGCTCTGTTACCGCCGAAATTGCACC 3' | 140 |
| F396V | F396VFw | 5'GATAGCTCTGTTGTTGCCGAAATTGCACC 3' | 141 |
| F396W | F396WFw | 5'GATAGCTCTGTTGGCGCCGAAATTGCACC 3' | 142 |
| F396Y | F396YFw | 5'GATAGCTCTGTTTACGCCGAAATTGCACC 3' | 143 |

**Table 6: Reverse primer for F396**

| Amino acid substitution | Primer name | Sequence | SEQ ID NO. |
|---|---|---|---|
| F396A | F396ARv | 5'GGTGCAATTTCGGCCGCAACAGAGCTATC 3' | 144 |
| F396C | F396CRv | 5'GGTGCAATTTCGGCGCAAACAGAGCTATC 3' | 145 |
| F396D | F396DRv | 5'GGTGCAATTTCGGCGTCAACAGAGCTATC 3' | 146 |
| F396E | F396ERv | 5'GGTGCAATTTCGGCTTCAACAGAGCTATC 3' | 147 |
| F396G | F396GRv | 5'GGTGCAATTTCGGCACCAACAGAGCTATC 3' | 148 |
| F396H | F396HRv | 5'GGTGCAATTTCGGCGTGAACAGAGCTATC 3' | 149 |
| F396I | F396IRv | 5'GGTGCAATTTCGGCGATAACAGAGCTATC 3' | 150 |
| F396K | F396KRv | 5'GGTGCAATTTCGGCTTTAACAGAGCTATC 3' | 151 |
| F396L | F396LRv | 5'GGTGCAATTTCGGCCAGAACAGAGCTATC 3' | 152 |
| F396M | F396MRv | 5'GGTGCAATTTCGGCCATAACAGAGCTATC 3' | 153 |
| F396N | F396NRv | 5'GGTGCAATTTCGGCGTTAACAGAGCTATC 3' | 154 |
| F396P | F396PRv | 5'GGTGCAATTTCGGCCGGAACAGAGCTATC 3' | 155 |
| F396Q | F396QRv | 5'GGTGCAATTTCGGCCTGAACAGAGCTATC 3' | 156 |
| F396R | F396RRv | 5'GGTGCAATTTCGGCGAAAACAGAGCTATC 3' | 157 |
| F396S | F396SRv | 5'GGTGCAATTTCGGCAGAAACAGAGCTATC 3' | 158 |

(continued)

| Amino acid substitution | Primer name | Sequence | SEQ ID NO. |
|---|---|---|---|
| F396T | F396TRv | 5'GGTGCAATTTCGGCGGTAACAGAGCTATC 3' | 159 |
| F396V | F396VRv | 5'GGTGCAATTTCGGCAACAACAGAGCTATC 3' | 160 |
| F396W | F396WRv | 5'GGTGCAATTTCGGCGCCAACAGAGCTATC 3' | 161 |
| F396Y | F396YRv | 5'GGTGCAATTTCGGCGTAAACAGAGCTATC 3' | 162 |

## Example 3: Analysis of enzymatic activity of mutant ChOx

Mutant ChOx was produced using the mutant ChOx expression plasmid obtained in Example 2, and studied for its enzymatic activity.

(1) Culture

*Escherichia coli* strain BL21 (DE3) was transformed with the wild-type ChOx expression plasmid prepared in Example 1 or the mutant ChOx expression plasmid prepared in Example 2. These transformants were separately shake-cultured at 37°C for 12 hours in 3 mL of an LB medium (containing 50 $\mu$g/mL kanamycin) using an L-shaped tube. 1 mL each of these culture solutions was inoculated to a 500-mL Erlenmeyer flask with a baffle containing 100 mL of an LB medium (containing 50 $\mu$g/mL kanamycin) and gyratory-cultured at 37°C. At the point in time when OD600 reached around 0.6, IPTG (isopropyl-$\beta$-D-thiogalactopyranoside) was added thereto at a final concentration of 1 mM, followed by culture at 20°C for 24 hours.

(2) Preparation of water-soluble fraction

From the culture solution thus cultured, bacterial cells were collected and washed. Then, the obtained wet bacterial cells were suspended in a 10 mM potassium phosphate buffer (pH 7.0) and sonicated. Then, the homogenate was centrifuged at 17400xg at 4°C for 20 minutes, and the supernatant was collected. This supernatant was further ultracentrifuged at 100400xg at 4°C for 60 minutes, and the supernatant was collected. The obtained supernatant was dialyzed against a 10 mM potassium phosphate buffer (pH 7.0), and this was used as a water-soluble fraction. This water-soluble fraction was used as a ChOx sample to determine cholesterol oxidase (ChOx) and cholesterol dehydrogenase (ChDH) activities for each of wild-type ChOx and mutant ChOx.

(4) Preparation of substrate solution

Cholesterol powder was dissolved at a concentration of 100 mM in Triton X-100 and incubated at 80°C to completely dissolve cholesterol. The 100 mM cholesterol solution was diluted 10-fold with pure water, cooled in running water, and brought to room temperature. Then, sodium cholate was added thereto at a final concentration of 3 mM to prepare a 10 mM cholesterol solution. For activity determination, the cholesterol solution was appropriately diluted with pure water to prepare various concentrations of substrate solutions.

(5) Determination of ChOx activity

The ChOx activity was determined by determining change in absorbance at 546 nm over time derived from a dye generated using peroxidase, a Trinder reagent (TODB), and 4-aminoantipyrine from hydrogen peroxide generated through reaction with the substrate. The reaction was performed under conditions shown below.

The reaction was initiated by adding the substrate to a reaction solution (10 mM potassium phosphate buffer pH 7.0 + 15 mM 4-aminoantipyrine + 1.5 mM TODB + 2 U/ml peroxidase; all the concentrations are final concentrations) containing the enzyme solution, and change in absorbance at 546 nm was determined. Various concentrations of cholesterol were used as the substrate. The amount of an enzyme that forms 1 $\mu$mol hydrogen peroxide for 1 minute is defined as 1 U. 38 mM-1 cm$^{-1}$ was used as the molar absorption coefficient of TODB at pH 7.0. The formula for calculating an activity value from change in absorbance is shown below.

$$U/ml = \Delta ABS_{546}/min \times 2/38 \times 10$$

$$U/mg = U/ml/protein\ mg/ml$$

(6) Determination of ChDH activity

The ChDH activity was determined by quantifying change in absorbance at 600 nm over time derived from the fading of DCIP reduced through reaction with the substrate. The reaction was performed under conditions shown below.
The reaction was initiated by adding the substrate to a reaction solution (10 mM potassium phosphate buffer pH 7.0 + 0.6 mM PMS + 0.06 mM DCIP; all the concentrations are final concentrations) containing the enzyme solution, and change in absorbance at 600 nm was determined. Those used in the ChOx activity determination were used as the substrate. The amount of an enzyme that reduces 1 $\mu$mol DCIP is defined as 1 U. The activity value was calculated according to the formula shown below. 16.3 mM-1 cm$^{-1}$ was used as the molar absorption coefficient of DCIP at pH 7.0.

$$U/ml = \Delta ABS_{600}/min \times 1/16.3 \times 5$$

$$U/mg = U/ml/protein\ mg/ml$$

The results of activity determination of the wild-type ChOx and the mutant ChOx are shown in Tables 7 to 9.
The oxidase activities of all M159 mutant enzymes were largely reduced. Among them, M159F, M159L, and M159V had dehydrogenase activity improved to 1.7 to 2.9 times the wild-type. Particularly, M159F had an oxidase activity value of $2.0 \times 10^{-2}$ U/mg and a dehydrogenase activity value of $2.2 \times 10^{-2}$ U/mg, which was 2.9 times the wild-type. The ratio of the dehydrogenase activity to the oxidase activity was 0.28% in the wild-type, whereas this ratio was 110% in M159F, which was improved to approximately 390 times the wild-type.
The oxidase activities of all the V228 mutant enzymes were lower than the wild-type (2.7 U/mg). The mutant that exhibited the lowest activity was V228D ($2.0 \times 10^{-4}$ U/mg). This value was approximately 1/10000 of the wild-type, showing significantly reduced reactivity to oxygen. In addition to V228D, V228N, V228Q, V228S, and V228K exhibited an oxidase activity value as very low as 1% or less of the wild-type (V228N: $7.0 \times 10^{-3}$ U/mg, V228E: $5.0 \times 10^{-3}$ U/mg, V228S: $9.0 \times 10^{-3}$ U/mg, V228K: $8.8 \times 10^{-3}$ U/mg). On the other hand, the Val228 mutants having substitution to isoleucine, leucine, or phenylalanine, including V228A, had a relatively high oxidase activity value (ratio to wild-type: 10%-) and thus retained reactivity to oxygen (V228I: 2.2 U/mg, V228L: $9.4 \times 10^{-1}$ U/mg, V228F: $2.2 \times 10^{-1}$ U/mg). 8 mutants having dehydrogenase activity improved compared with the wild-type were obtained. Among them, V228T exhibited activity ($5.6 \times 10^{-2}$ U/mg) approximately 5 times the wild-type. In addition, the mutants having substitution to lysine, serine, or cysteine exhibited high dehydrogenase activity (V228K: $3.4 \times 10^{-2}$ U/mg, V228C: $3.1 \times 10^{-2}$ U/mg, V228S: $2.0 \times 10^{-2}$ U/mg). On the other hand, the mutants having substitution to leucine or isoleucine had activity reduced to 1/10 of the wild-type. (V228L: $1.0 \times 10^{-3}$ U/mg, V228I: $1.0 \times 10^{-3}$ U/mg). V228D and V228R had no detectable dehydrogenase activity.
The oxidase activities of the F396 mutant enzymes were reduced. The F396W mutant enzyme had an oxidase activity value of $1.2 \times 10^{-1}$ U/mg and a dehydrogenase activity value of $2.0 \times 10^{-2}$ U/mg. The oxidase activity was reduced compared with that of the wild-type ChOx, and the dehydrogenase activity was improved to twice or more the wild-type. The ratio of the dehydrogenase activity to the oxidase activity was 16%, which was 57 times the wild-type (0.28%). F396N and F396D had not only oxidase activity reduced to 1.9% and 0.032%, respectively, of the wild-type but also dehydrogenase activity reduced to 23% and 14%, respectively, of the wild-type. Each of the mutant enzymes F396M, F396L, F396V, F396I, and F396Y maintained oxidase activity (30 to 80% of the wild-type) compared with the other mutant enzymes.

**Table 7:**

|  | Oxidase activity (U/mg) | Dehydrogenase activity (U/mg) | Dh/Ox (%) |
|---|---|---|---|
| WT | 2.8 (100%) | $7.7 \times 10^{-3}$ (100%) | 0.28 |
| M159A | $3.6 \times 10^{-2}$ (1.3%) | $6.4 \times 10^{-3}$ (83%) | 18 |
| M159C | $2.1 \times 10^{-2}$ (0.75%) | $7.7 \times 10^{-3}$ (100%) | 37 |
| M159D | $7.4 \times 10^{-4}$ (0.026%) | $1.6 \times 10^{-3}$ (21%) | 220 |
| M159E | $4.4 \times 10^{-4}$ (0.016%) | $1.0 \times 10^{-3}$ (13%) | 230 |

(continued)

|  | Oxidase activity (U/mg) | Dehydrogenase activity (U/mg) | Dh/Ox (%) |
|---|---|---|---|
| M159F | $2.0 \times 10^{-2}$ (0.71%) | $2.2 \times 10^{-2}$ (290%) | 110 |
| M159G | $1.4 \times 10^{-3}$ (0.05%) | $1.0 \times 10^{-3}$ (13%) | 71 |
| M159H | $6.2 \times 10^{-3}$ (0.22%) | $7.6 \times 10^{-4}$ (9.9%) | 12 |
| M159I | $3.1 \times 10^{-1}$ (11%) | $6.4 \times 10^{-3}$ (83%) | 2.1 |
| M159K | $6.0 \times 10^{-4}$ (0.021%) | $3.0 \times 10^{-3}$ (39%) | 500 |
| M159L | $5.0 \times 10^{-1}$ (18%) | $1.6 \times 10^{-2}$ (210%) | 3.2 |
| M159N | $5.2 \times 10^{-2}$ (1.9%) | $4.2 \times 10^{-4}$ (5.5%) | 0.81 |
| M159P | $1.9 \times 10^{-3}$ (0.068%) | $4.0 \times 10^{-4}$ (5.2%) | 21 |
| M159Q | $3.1 \times 10^{-2}$ (1.1%) | $5.0 \times 10^{-3}$ (65%) | 16 |
| M159R | $1.5 \times 10^{-4}$ (0.0054%) | $4.8 \times 10^{-4}$ (6.2%) | 320 |
| M159S | $1.8 \times 10^{-1}$ (6.4%) | $2.9 \times 10^{-3}$ (38%) | 1.6 |
| M159T | $2.9 \times 10^{-1}$ (10%) | $2.9 \times 10^{-3}$ (38%) | 1 |
| M159V | $4.7 \times 10^{-2}$ (1.7%) | $1.3 \times 10^{-2}$ (170%) | 28 |
| M159W | $2.3 \times 10^{-2}$ (0.82%) | $3.8 \times 10^{-4}$ (4.9%) | 1.7 |
| M159Y | $7.7 \times 10^{-3}$ (0.28%) | $5.0 \times 10^{-3}$ (65%) | 65 |

**Table 8:**

|  | Oxidase activity (U/mg) | Dehydrogenase activity (U/mg) | Dh/Ox (%) |
|---|---|---|---|
| WT | 2.5 - 2.9 | $7.0 \times 10^{-3}$ - $10 \times 10^{-3}$ | 0.25-0.35% |
| V228A | $3.0 \times 10^{-2}$ - $4.3 \times 10^{-2}$ | $5.7 \times 10^{-2}$ - $7.4 \times 10^{-2}$ | 150-180% |
| V228C | $7.0 \times 10^{-2}$ | $3.1 \times 10^{-2}$ | 44% |
| V228D | $2.0 \times 10^{-4}$ | not detected | - |
| V228E | $5.0 \times 10^{-3}$ | $1.4 \times 10^{-2}$ | 280% |
| V228F | $2.2 \times 10^{-1}$ | $5.0 \times 10^{-3}$ | 2.3% |
| V228G | $8.0 \times 10^{-3}$ | $1.8 \times 10^{-2}$ | 225% |
| V228H | $1.0 \times 10^{-2}$ | $6.0 \times 10^{-3}$ | 60% |
| V228I | 2.2 | $1.0 \times 10^{-3}$ | 0.06% |
| V228K | $8.8 \times 10^{-3}$ | $3.4 \times 10^{-2}$ | 374% |
| V228L | $9.4 \times 10^{-1}$ | $1.0 \times 10^{-3}$ | 0.1% |
| V228M | $2.2 \times 10^{-1}$ | $6.0 \times 10^{-4}$ | 0.27% |
| V228N | $7.0 \times 10^{-3}$ | $9.0 \times 10^{-3}$ | 130% |
| V228P | $4.8 \times 10^{-2}$ | $1.2 \times 10^{-2}$ | 25% |
| V228Q | $2.9 \times 10^{-2}$ | $6.7 \times 10^{-3}$ | 23% |
| V228R | $2.2 \times 10^{-3}$ | not detected | - |
| V228S | $9.0 \times 10^{-3}$ | $2.0 \times 10^{-2}$ | 222% |
| V228T | $1.7 \times 10^{-1}$ | $5.6 \times 10^{-2}$ | 33% |
| V228W | $4.9 \times 10^{-2}$ | $6.2 \times 10^{-3}$ | 13% |

(continued)

|  | Oxidase activity (U/mg) | Dehydrogenase activity (U/mg) | Dh/Ox (%) |
|---|---|---|---|
| V228Y | $1.2 \times 10^{-1}$ | $1.3 \times 10^{-2}$ | 110% |

**Table 9:**

|  | Oxidase activity (U/mg) | Dehydrogenase activity (U/mg) | Dh/Ox (%) |
|---|---|---|---|
| WT | 2.8 (100%) | $7.7 \times 10^{-3}$ (100%) | 0.28 |
| F396A | $5.9 \times 10^{-2}$ (2.1%) | $1.8 \times 10^{-3}$ (23%) | 3.1 |
| F396C | $2.2 \times 10^{-1}$ (7.9%) | $2.3 \times 10^{-3}$ (30%) | 1.0 |
| F396D | $8.9 \times 10^{-4}$ (0.032%) | $1.1 \times 10^{-3}$ (14%) | 120 |
| F396E | $2.5 \times 10^{-3}$ (0.089%) | not detected | - |
| F396G | $4.9 \times 10^{-1}$ (18%) | $8.3 \times 10^{-4}$ (11%) | 0.17 |
| F396H | $5.8 \times 10^{-2}$ (2.1%) | $1.5 \times 10^{-3}$ (19%) | 2.6 |
| F396I | $9.7 \times 10^{-1}$ (35%) | $2.3 \times 10^{-3}$ (30%) | 0.24 |
| F396K | $8.9 \times 10^{-2}$ (3.2%) | $3.0 \times 10^{-3}$ (39%) | 3.4 |
| F396L | 1.1 (39%) | $2.6 \times 10^{-3}$ (34%) | 0.24 |
| F396M | $8.4 \times 10^{-1}$ (30%) | $5.9 \times 10^{-3}$ (77%) | 0.70 |
| F396N | $5.3 \times 10^{-2}$ (1.9%) | $1.8 \times 10^{-3}$ (23%) | 3.4 |
| F396P | $2.1 \times 10^{-4}$ (0.0075%) | not detected | - |
| F396Q | $9.6 \times 10^{-4}$ (0.034%) | not detected | - |
| F396R | $3.3 \times 10^{-3}$ (0.12%) | not detected | - |
| F396S | $8.5 \times 10^{-2}$ (3.0%) | $4.1 \times 10^{-3}$ (53%) | 2.7 |
| F396T | $1.9 \times 10^{-1}$ (6.8%) | $3.1 \times 10^{-3}$ (40%) | 1.7 |
| F396V | $7.6 \times 10^{-1}$ (27%) | $2.4 \times 10^{-3}$ (31%) | 0.32 |
| F396W | $1.2 \times 10^{-1}$ (4.3%) | $2.0 \times 10^{-2}$ (260%) | 16 |
| F396Y | 1.7(61%) | $9.1 \times 10^{-3}$ (120%) | 0.54 |

Tables 10-12 show alignment of the amino acid sequences that are annotated to be cholesterol oxidases. The entire sequences of these mutated cholesterol oxidases are set forth in SEQ ID NOs: 1-48. Alignment was created using AlignX application of Vector NTI suite 6.0. Those skilled in the art will appreciate that another alignment software programs such as Blast will provide the same or substantially the same alignment.

It is evident from Table 10 that Met159 of SEQ ID NO:1 is conserved among the amino acid sequences listed in Table 10. Accordingly, a person skilled in the art can easily identify the Met or Ile residue corresponding to the Met159 of SEQ ID NO:1 within the conserved region using any of commercially available software programs for sequence alignment, and understand that a mutant cholesterol oxidase is easily prepared by introducing modification on that Met or Ile residue.

**Table 10:**

| Origin* | position of mutation |  |  |  | SEQ ID NO** |
|---|---|---|---|---|---|
| sp\|P12676 | M159 | 149 | VGGGSLVNGG**M**AVEPKRSYFE | 169 | 1 |
| gb\|ABS32193 | M155 | 145 | VGGGSLVNGG**M**AVAPKRSYFE | 165 | 2 |
| ref\|NP_821583 | M160 | 150 | YGGGSLVNGG**M**AVTPRRSYFE | 170 | 3 |
| emb\|GAC20926 | M162 | 152 | VGGGSLVNGG**M**AVVPKRSYFE | 172 | 4 |

(continued)

| Origin* | position of mutation | | | | SEQ ID NO** |
|---|---|---|---|---|---|
| gb|ADX66466 | M165 | 155 | VGGGSLVNGG**M**AVVPKRSYFE | 175 | 5 |
| gb|AAR16516 | M162 | 152 | VGGGSLVNGG**M**AVVPKRSYFE | 172 | 6 |
| gb|AAZ66744 | M165 | 155 | VGGGSLVNGG**M**AVVPKRAYFE | 175 | 7 |
| gb|AAA69655 | M160 | 150 | VGGGSLVNGG**M**AVTPRRSYFQ | 170 | 8 |
| ref|ZP_07282326 | M157 | 147 | VGGGSLVNGA**M**AVQPKRSYFE | 167 | 9 |
| ref|ZP_07286354 | M165 | 155 | VGGGSLVNGG**M**APTPRRSYFA | 175 | 10 |
| ref|ZP_04998002 | M166 | 156 | VGGGSLVNGG**M**SPTPRRSYFS | 176 | 11 |
| ref|YP_003492288 | M154 | 144 | VGGGSLVNGS**M**AVTPLRSYFA | 164 | 12 |
| gb|AD109201 | M158 | 148 | VGGGSLVNGG**M**AVTPSRAYFQ | 168 | 13 |
| ref|ZP_07603147 | I164 | 154 | VGGGSLVNGG**I**AVTPSRSYFQ | 174 | 14 |
| ref|YP_003512290 | M148 | 138 | VGGGSLVNGG**M**AVVPRRKYFQ | 158 | 15 |
| ref|ZP_05008217 | M154 | 144 | VGGGSLVNGG**M**AVTPRRPYFS | 164 | 16 |
| ref|ZP_06770826 | M168 | 158 | VGGGSLVNGG**M**AVTPRRPYFS | 178 | 17 |
| ref|ZP_06566730 | M151 | 141 | VGGGSLVNGG**M**AVTPRRGYFE | 161 | 18 |
| ref|NP_827244 | M159 | 149 | VGGGSLVNGG**M**AVTPLQSYFA | 169 | 19 |
| ref|YP_001108512 | M139 | 129 | VGGGSLVNGG**M**AVTPRRGYFE | 149 | 20 |
| ref|ZP_07292951 | I161 | 151 | VGGGSLVNGG**I**AVTPPRAYFQ | 171 | 21 |
| ref|ZP_07303187 | M159 | 149 | VGGGSLVNGG**M**AVTPLRSYFA | 169 | 22 |
| ref|ZP_01689718 | M146 | 136 | VGGGSLVNGG**M**AVTPPMNYFQ | 156 | 23 |
| ref|YP_003384280 | M157 | 147 | VGGGSLVNGG**M**APTPRRSYFE | 167 | 24 |
| ref|ZP_06916507 | M159 | 149 | VGGGSLVNGS**M**AVTPLQSYFA | 169 | 25 |
| ref|YP_003134867 | M150 | 140 | VGGGSLVNGA**M**AVTPKRATFA | 160 | 26 |
| emb|CAC44897 | M167 | 157 | VGGGSLVNGG**M**AVTPKRNYFE | 177 | 27 |
| pdb|1COY_A | M122 | 112 | VGGGSLVNGG**M**AVTPKRNYFE | 132 | 28 |
| sp|P22637 | M167 | 157 | VGGGSLVNGG**M**AVTPKRNYFE | 177 | 29 |
| ref|ZP_06830857 | M167 | 157 | VGGGSLVNGG**M**AVTPKRNYFE | 177 | 30 |
| gb|ABC75776 | M167 | 157 | VGGGSLVNGG**M**AVTPKRNYFE | 177 | 31 |
| gb|ABG24169 | M161 | 157 | VGGGSLVNGG**M**AVTPKRNYFE | 177 | 32 |
| emb|CAZ68116 | M158 | 148 | VGGGSLVNGG**M**AVTPKRNYFE | 168 | 33 |
| ref|YP_002764459 | M167 | 157 | VGGGSLVNGG**M**AVTPKRNYFE | 177 | 34 |
| ref|ZP_04388109 | M137 | 127 | VGGGSLVNGG**M**AVTPKRNYFE | 147 | 35 |
| ref|YP_003769621 | M142 | 132 | VGGGSLVNGG**M**AVTPKRENFG | 152 | 36 |
| ref|YP_003339891 | M166 | 156 | VGGGSLVNGG**M**AVTPKRENFG | 176 | 37 |
| ref|ZP_07290670 | M168 | 158 | VGGGSLVNGG**M**AVTPKRQNFA | 178 | 38 |
| ref|YP_003116660 | M165 | 155 | IGGGSLVNGG**M**AVTPKQENFG | 175 | 39 |
| ref|ZP_06588880 | M169 | 159 | VGGGSLVNGG**M**AVTPRRENFG | 179 | 40 |
| ref|ZP_04697978 | M121 | 111 | VGGGSLVNGG**M**AVTPRRENFG | 131 | 41 |
| ref|ZP_06276136 | M166 | 156 | VGGGSLVNGG**M**AVTPRRENFG | 176 | 42 |

(continued)

| Origin* | position of mutation | | | | SEQ ID NO** |
|---------|---------------------|-----|---------------------------|-----|-------------|
| ref\|YP_001821989 | M166 | 156 | VGGGSLVNGG**M**AVTPRRENFG | 176 | 43 |
| ref\|ZP_05002034 | M165 | 155 | VGGGSLVNGG**M**AVTPKRERFG | 175 | 44 |
| ref\|ZP_06907496 | M166 | 156 | VGGGSLVNGG**M**AVTPRRENFG | 176 | 45 |
| ref\|YP_003100211 | M163 | 153 | VGGGSLVNGG**M**AVTPRRERFA | 173 | 46 |
| ref\|ZP_07085639 | M142 | 132 | VGGGSLVNGG**M**AVTPKESYFR | 152 | 47 |
| gb\|ADX68765 | M139 | 129 | VGGGSLVNGG**M**AVLPKKNYFK | 149 | 48 |
| * Databases: gb: GenBank; sp: Swissprot; ref: RefSeq; emb: EMBL; pdb: Protein Data Bank<br>** SEQ ID NOs represent the full length sequence | | | | | |

It is evident from Table 11 that Val228 of SEQ ID NO:1 is conserved among the amino acid sequences listed in Table 11. Accordingly, a person skilled in the art can easily identify the Val, Met or Ile residue corresponding to the Val228 of SEQ ID NO:1 within the conserved region using any of commercially available software programs for sequence alignment, and understand that a mutant cholesterol oxidase is easily prepared by introducing modification on that Val, Met or He residue.

**Table 11:**

| Origin* | position of mutation | | | | SEQ ID NO** |
|---------|---------------------|-----|----------------------------|-----|-------------|
| sp\|P12676 | V228 | 218 | AGKAGLGTVF_V_PNVYDFGYMQ | 238 | 1 |
| gb\|ABS32193 | V224 | 214 | AQKAGLGTVH_V_PNVYDFDHMR | 234 | 2 |
| ref\|NP_821583 | V229 | 219 | ASNAGLSTTF_Y_PNVYPNVYDWDYMR | 239 | 3 |
| emb\|AC20926 | V231 | 221 | AGKAGLGTTF_V_PNVYDFDYMR | 241 | 4 |
| gb\|ADX66466 | V234 | 224 | AIGKAGLSTTF_V_PNVYDFDYMR | 244 | 5 |
| gb\|AAR16516 | V231 | 221 | AGKAGLSTTF_V_PNVYDFDHMR | 241 | 6 |
| gb\|AAZ66744 | V234 | 224 | ASKAGLGTTF_V_PNVYDFGHMR | 244 | 7 |
| gb\|AAAB9655 | V229 | 219 | AENAGLKTTF_V_PNVYDWDYMR | 239 | 8 |
| ref\|ZP_07282326 | V226 | 216 | AQKTGLKTTFVPS_V_YDFEYMK | 236 | 9 |
| ref\|ZP'8 07286354 | V234 | 224 | AQNTGLKTTFVPN_V_YDFGYMK | 244 | 10 |
| ref\|ZP_04998002 | V235 | 225 | AQNTGLKTVF_V_PNVYDFEYMK | 245 | 11 |
| ref\|YP_003492288 | V223 | 213 | AAKAGLRTTF_V_YPSVYDFDHMQ | 233 | 12 |
| gb\|ADI09201 | V227 | 217 | AKTAGLKTVF_V_PNVYDFDYMQ | 237 | 13 |
| ref\|ZP_7603147 | M233 | 223 | AARAGLKTVF_M_PNVYDFDYMR | 243 | 14 |
| ref\|YP_003512290 | V217 | 207 | AEETGLATTF_V_PNVYDFDHMA | 227 | 15 |
| ref\|ZP_05008217 | V223 | 213 | AARAGLGTVF_V_PNVYDFDYMR | 233 | 16 |
| ref\|ZP_46770826 | V237 | 227 | AARAGLGTVF_V_PNVYDFDYMR | 247 | 17 |
| ref\|ZP_06566730 | V220 | 210 | AQRAGLRTTF_V_PNVYDFGYMR | 230 | 18 |
| ref\|NP_827244 | V228 | 218 | ATNTGLKTTF_V_PNVYDFGYMQ | 238 | 19 |
| ref\|VP_001108512 | V208 | 198 | AGRAGLRTTF_V_PNVYDFGYMR | 218 | 20 |
| ref\|ZP_7292951 | V230 | 220 | ASAAGLKTVFVPS_V_YDFDYMR | 240 | 21 |
| ref\|ZP_07303187 | V228 | 218 | ADNAGLKTTF_V_PNVYDFGHME | 238 | 22 |
| ref\|ZP_01689718 | V215 | 205 | AEKAGFKTVT_V_PNIYDYNYMQ | 225 | 23 |

(continued)

| Origin* | position of mutation | | | | SEQ ID NO** |
|---|---|---|---|---|---|
| ref\|YP_003384280 | 1226 | 216 | AHQAGFRTAVIPNVYDFGYLE | 236 | 24 |
| ref\|ZP_06916507 | V228 | 218 | AQNTGLKTTFVSVYDFGYMQ | 238 | 25 |
| ref\|YP_003134867 | V219 | 209 | AHNAGLTTTFVPSVYDFARMR | 229 | 26 |
| emb\|CAC44897 | V236 | 226 | AQRSGFTTAFVPNVYDFEYMK | 246 | 27 |
| pdb\|1C0Y_A | V191 | 181 | AQRSGFTTAFVPNVYDFEYMK | 201 | 28 |
| sp\|P22637 | V236 | 226 | AQRSGFTTAFVPNVYDFEYMK | 246 | 29 |
| ref\|ZP_06830857 | V236 | 226 | AQRSGFTTAFVPNVYDFEYMK | 246 | 30 |
| gb\|ABC75776 | V236 | 226 | AQRSGFTTAFVPNVYDFEYMK | 246 | 31 |
| gb\|ABG24169 | V236 | 226 | AQRSGFTTAFVPNVYDFEYMK | 246 | 32 |
| emb\|CAZ68116 | V227 | 217 | AQRSGFTTAFVPNVYDFEYMK | 237 | 33 |
| refl\|ZP_002764459 | V236 | 226 | AERSGYTTTFVPNVYDFNYMK | 246 | 34 |
| ref\|ZP_04388109 | V206 | 196 | AERSGYTTTFVPNVYDFNYMK | 216 | 35 |
| ref\|VP_003769621 | V211 | 201 | AQRSGFPFVFVPDVYDWDYME | 221 | 36 |
| ref\|YP_003339891 | V235 | 225 | AQRSGFPFVFVPDVYDWDYMK | 245 | 37 |
| ref\|ZP_07290674 | V237 | 227 | AQRSGFPFVFVPDVYDWDYMK | 247 | 38 |
| ref\|YP_003116660 | V234 | 224 | AGRSGFPFQFVPDVYDWNYMQ | 244 | 39 |
| ref\|ZP_06588880 | V238 | 228 | AQRSGFPFLFVPAVYDWDYMK | 248 | 40 |
| ref\|ZP_04697978 | V190 | 180 | AQRSGFPFLFVPAVYDWDYMK | 200 | 41 |
| ref\|ZP_06276136 | V235 | 225 | AQRSGFPFLFVPAVYDWDYMK | 245 | 42 |
| ref\|YP_001821989 | V235 | 225 | AQRSGFPFLFVPAVYDWDYMK | 245 | 43 |
| ref\|ZP_05002034 | V234 | 224 | AQRSGFPFVFVPNVYMEYMK | 244 | 44 |
| ref\|ZP_06907496 | V235 | 225 | AERSGFPFVLVPGVYDWDYLE | 245 | 45 |
| ref\|YP_003100211 | V232 | 222 | AQRSGFPFELVPGVYDWAHLE | 242 | 46 |
| ref\|ZP_07085639 | V211 | 201 | AHKAGFKTIRVPNVYDFKYME | 221 | 47 |
| gb\|ADX68765 | V208 | 198 | AQKAGYKTIRVPNVYNFKYME | 218 | 48 |
| * Databases: gb: GenBank; sp: Swissprot; ref: RefSeq; emb: EMBL; pdb: Protein Data Bank  \*\* SEQ ID NOs represent the full length sequence | | | | | |

It is evident from Table 12 that Phe396 of SEQ ID NO: 1 is conserved among the amino acid sequences listed in Table 12, Accordingly, a person skilled in the art can easily identify the Phe residue corresponding to the Phe396 of SEQ ID NO:1 within the conserved region using any of commercially available software programs for sequence alignment, and understand that a mutant cholesterol oxidase is easily prepared by introducing modification on that Phe residue.

**Table 12:**

| Origin* | position of mutation | | | | SEQ ID NO** |
|---|---|---|---|---|---|
| sp\|P12676 | F396 | 386 | DAWDN----SDSSVFAEIAPMPAGL | 406 | 1 |
| gb\|ABS32193 | F392 | 382 | DDWDN----PONPVFAEIAPMPAGL | 402 | 2 |
| ref\|NP_821583 | F397 | 387 | DDWDN----PDTPVFAEIAPLPAGV | 407 | 3 |
| emb\|CAC20926 | F399 | 389 | DDMNN----PTAPVFAEIAPMPAGL | 409 | 4 |
| gb\|ADX66466 | F402 | 392 | DDWDN----PAAPVFAEIAPMPAGL | 412 | 5 |

(continued)

| Origin* | position of mutation | | | | SEQ ID NO** |
|---|---|---|---|---|---|
| gb\|AAR16516 | F399 | 389 | DDWDN----PAAPVFAEIAPMPAGL | 409 | 6 |
| gb\|AAZ66744 | F402 | 392 | DDWNN----AAAPVFAEIAPMPAGA | 412 | 7 |
| gb\|AAA69655 | F397 | 387 | DDWDN----PDNPVFAEIAPMPAGL | 407 | 8 |
| ref\|ZP_7282326 | F394 | 384 | DDWDN----PAHPVFAEIAPVPAGL | 404 | 9 |
| ref\|ZP_07286354 | F402 | 392 | DDWDN----AANPVFAEIAPLPMGI | 412 | 10 |
| ref\|ZP_04998002 | F403 | 393 | DDWDN----AANPVFIEIAPLPMGF | 413 | 11 |
| ref\|YP_003492288 | F391 | 381 | DDWAN----TANPVFAEIAPLPTGL | 401 | 12 |
| gb\|AD109201 | F395 | 385 | DDWSN----ATNPYFAEIAPLPAGT | 405 | 13 |
| ref\|ZP_07603147 | F401 | 391 | DDWSN----TANPVFAEIAPLPAGL | 411 | 14 |
| ref\|YP_003512290 | F385 | 375 | DDWDN----EAARVFAEIAPVPAGF | 395 | 15 |
| ref\|ZP_05008217 | F397 | 387 | DDWNN----PTHPVFAEIAPLPMGL | 407 | 16 |
| ref\|ZP_06770826 | F411 | 401 | DDWNN----PTHPVFAEIAPLPMGL | 421 | 17 |
| ref\|ZP_06566730 | F388 | 378 | DNWDD----PVHPVFAEIAPLPAGL | 398 | 18 |
| ref\|NP_827244 | F396 | 386 | DDWAN----TSNPVFAEIAPLPMGL | 406 | 19 |
| ref\|YP_00118512 | F376 | 366 | DNWDD----PVHPVFAEIAPLPAGL | 386 | 20 |
| ref\|ZP_07292951 | F398 | 388 | DDWSN----AANPVFAEIAPLPAGT | 408 | 21 |
| ref\|ZP_07303187 | F396 | 386 | DDWAN----TANPVFAEIAPLPMGL | 406 | 22 |
| ref\|ZP_01689718 | F383 | 373 | NDWDN----ASNPVFAEIAPLPTGF | 393 | 23 |
| ref\|YP_003384280 | F393 | 383 | DNWDD----PVHPAFAEIAPLPTGL | 403 | 24 |
| ref\|ZP_06916507 | F396 | 386 | DDWAN----TDNPVFAEIAPLPTGL | 406 | 25 |
| ref\|YP_003134867 | F387 | 377 | DAWDD----PRHPVFAEVAPMPAGV | 397 | 26 |
| emb\|CAC44897 | F404 | 394 | DNWAD----PTAPIFAEIAPLPAGL | 414 | 27 |
| pdb\|1C0Y_A | F359 | 349 | DNWAD----PTAPIFAEIAPLPAGL | 369 | 28 |
| sp\|P22637 | F404 | 394 | DNWAD----PTAPIFAEIAPLPAGL | 414 | 29 |
| ref\|ZP_06830857 | F404 | 394 | DNWAD----PTAPIFAEIAPLPAGL | 414 | 30 |
| gb\|ABC75776 | F404 | 394 | DNWAD----PAAPIFAEIAPLPAGL | 414 | 31 |
| gb\|ABG24169 | F405 | 395 | DNWAD----PTAPIFAEIAPLPAGL | 415 | 32 |
| emb\|CAZ68116 | F395 | 385 | DMWAD----PTAPIFAEIAPLPAGL | 405 | 33 |
| ref\|YP_002764459 | F405 | 395 | DNWAD----TSAPVFAEIAPFPAGT | 415 | 34 |
| ref\|ZP_04388109 | F375 | 365 | DNWAD----TSAPVFAEIAPFPAGT | 385 | 35 |
| ref\|YP_003769621 | F377 | 369 | DNWAA----GGA----FAEVAPLPTGI | 387 | 36 |
| ref\|YP_003339891 | F401 | 393 | DNWAA----GGA----FAEVAPLPTGI | 411 | 37 |
| ref\|ZP_07290670 | F403 | 395 | DNWDA----66A----FAEVAPLPTGI | 413 | 38 |
| ref\|YP_003116660 | F400 | 392 | DNWTK----GGA----FAEVAPLPIGI | 410 | 39 |
| ref\|ZP_06588880 | F404 | 396 | DNWDA----GGA----FAEIAPLPTGI | 414 | 40 |
| ref\|ZP_04697978 | F356 | 348 | DNWDA----GGA----FAEIAPLPTGI | 366 | 41 |
| ref\|ZP_06276136 | F401 | 393 | DNWDA----GGA----FAEVAPLPTGI | 411 | 42 |
| ref\|YP_001821989 | F401 | 393 | DNWDA----GGA----FAEVAPLPT6I | 411 | 43 |

(continued)

| Origin* | position of mutation | | | | SEQ ID NO** |
|---|---|---|---|---|---|
| ref|ZP_05002034 | F400 | 392 | DNWDA----GGA----FAEVAPLPTGI | 410 | 44 |
| ref|ZP_06907496 | F401 | 393 | DNWQA----GGA----FAEVAPLPTGI | 411 | 45 |
| ref|YP_003100211 | F397 | 389 | DNWAA----GGA----FAEVAPLPTGV | 407 | 46 |
| ref|ZP_07086639 | F380 | 370 | DNWDD----PEHQFFTEIAPLPMGM | 390 | 47 |
| gb|ADX68765 | F377 | 367 | DNWDD----PKYPFFAEIAPLPMGM | 387 | 48 |
| * Databases: gb: GenBank; sp: Swissprot; ref: RefSeq; emb: EMBL; pdb: Protein Data Bank<br>** SEQ ID NOs represent the full length sequence | | | | | |

Industrial Applicability

The mutated cholesterol oxidase of the invention may be used for the measurement of cholesterol or lipoproteins associated with cholesterol, such as high density lipoprotein or low density lipoprotein, which is clinically useful in diagnosis and control of certain health conditions.

**Claims**

1. A mutant cholesterol oxidase consisting of an amino acid sequence set forth in SEQ ID NO: 1 and having an oxidase activity of less than its dehydrogenase activity, wherein the amino acid residue at position 159 is an amino acid residue selected from Phe, Leu, Val, Cys, Ile, Ala, Gln, Tyr, Lys, and Ser, the amino acid residue at position 228 is Val and the amino acid residue at position 396 is Phe.

2. The mutant cholesterol oxidase of claim 1, which has a reduced oxidase activity as compared to the wild-type cholesterol oxidase.

3. The mutant cholesterol oxidase of claim 1, which has the ratio of dehydrogenase/oxidase activity of about 2.0 or more.

4. The mutant cholesterol oxidase of claim 1, which has a dehydrogenase activity of 50% or more of the wild-type cholesterol oxidase.

5. An isolated polynucleotide encoding the mutant cholesterol oxidase of any one of claims 1-4.

6. A vector comprising the polynucleotide of claim 5.

7. A host cell transformed with a vector of claim 6.

8. A method for assaying cholesterol in a sample, comprising contacting the sample with the cholesterol oxidase of any one of claims 1-4, and measuring the amount of the cholesterol oxidized by the cholesterol oxidase.

9. A method for assaying HDL cholesterol comprising reacting HDL in a sample to generate cholesterol, contacting the cholesterol with the cholesterol oxidase of any one of claims 1-4, and measuring the amount of oxidized cholesterol.

10. A method for assaying LDL cholesterol comprising reacting LDL in a sample to generate cholesterol, contacting the cholesterol with the cholesterol oxidase of any one of claims 1-4, and measuring the amount of oxidized cholesterol.

11. A device for assaying cholesterol, HDL cholesterol, and/or LDL cholesterol in a sample comprising the cholesterol oxidase of any one of claims 1-4 and an electron mediator.

12. A kit for assaying cholesterol, HDL cholesterol, and/or LDL cholesterol in a sample comprising the cholesterol oxidase of any one of claims 1-4 and an electron mediator.

**13.** An enzyme electrode having the cholesterol oxidase of any one of claims 1-4 which is immobilized on the electrode.

**14.** An enzyme sensor for assaying cholesterol, HDL cholesterol, LDL cholesterol comprising the enzyme electrode of claim 13 as a working electrode.

**Patentansprüche**

**1.** Mutierte Cholesterinoxidase, bestehend aus einer Aminosäuresequenz gemäß SEQ ID NO: 1 und mit einer geringeren Oxidase-Aktivität als ihrer Dehydrogenase-Aktivität, wobei es sich bei dem Aminosäurerest an Position 159 um einen aus Phe, Leu, Val, Cys, Ile, Ala, Gln, Tyr, Lys und Ser ausgewählten Aminosäurerest, dem Aminosäurerest an Position 228 um Val und dem Aminosäurerest an Position 396 um Phe handelt.

**2.** Mutierte Cholesterinoxidase nach Anspruch 1, die eine reduzierte Oxidase-Aktivität im Vergleich zur Wildtyp-Cholesterinoxidase aufweist.

**3.** Mutierte Cholesterinoxidase nach Anspruch 1, die das Dehydrogenase/Oxidase-Aktivitätsverhältnis von etwa 2,0 oder mehr aufweist.

**4.** Mutierte Cholesterinoxidase nach Anspruch 1, die eine Dehydrogenase-Aktivität von 50% oder mehr der Wildtyp-Cholesterinoxidase aufweist.

**5.** Isoliertes Polynukleotid, codierend die mutierte Cholesterinoxidase nach einem der Ansprüche 1-4.

**6.** Vektor, umfassend das Polynukleotid nach Anspruch 5.

**7.** Wirtszelle, transformiert mit einem Vektor nach Anspruch 6.

**8.** Testverfahren für Cholesterin in einer Probe, umfassend Inkontaktbringen der Probe mit der Cholesterinoxidase nach einem der Ansprüche 1-4 und Messen der Menge des von der Cholesterin-oxidase oxidierten Cholesterins.

**9.** Testverfahren für HDL-Cholesterin, umfassend Umsetzen von HDL in einer Probe unter Erzeugung von Cholesterin, Inkontaktbringen des Cholesterins mit der Cholesterinoxidase nach einem der Ansprüche 1-4 und Messen der Menge an oxidiertem Cholesterin.

**10.** Testverfahren für LDL-Cholesterin, umfassend Umsetzen von LDL in einer Probe unter Erzeugung von Cholesterin, Inkontaktbringen des Cholesterins mit der Cholesterinoxidase nach einem der Ansprüche 1-4 und Messen der Menge an oxidiertem Cholesterin.

**11.** Testvorrichtung für Cholesterin, HDL-Cholesterin und/oder LDL-Cholesterin in einer Probe, umfassend die Cholesterinoxidase nach einem der Ansprüche 1-4 und einen Elektronenvermittler.

**12.** Testkit für Cholesterin, HDL-Cholesterin und/oder LDL-Cholesterin in einer Probe, umfassend die Cholesterinoxidase nach einem der Ansprüche 1-4 und einen Elektronenvermittler.

**13.** Enzymelektrode mit der Cholesterinoxidase nach einem der Ansprüche 1-4, die auf der Elektrode immobilisiert ist.

**14.** Enzymsensor zum Testen auf Cholesterin, HDL-Cholesterin, LDL-Cholesterin, umfassend die Enzym-elektrode nach Anspruch 13 als Arbeitselektrode.

**Revendications**

**1.** Cholestérol oxydase mutante constituée d'une séquence d'acides aminés présentée dans SEQ ID n° 1 et ayant une activité oxydase inférieure à son activité déshydrogénase, dans laquelle le résidu d'acide aminé en position 159 est un résidu d'acide aminé choisi parmi Phe, Leu, Val, Cys, Ile, Ala, Gln, Tyr, Lys et Ser, le résidu d'acide aminé en position 228 est Val et le résidu d'acide aminé en position 396 est Phe.

**2.** Cholestérol oxydase mutante selon la revendication 1, qui a une activité oxydase réduite par rapport à la cholestérol oxydase sauvage.

**3.** Cholestérol oxydase mutante selon la revendication 1, qui a le rapport d'activité déshydrogénase/oxydase d'environ 2,0 ou plus.

**4.** Cholestérol oxydase mutante selon la revendication 1, qui a une activité déshydrogénase de 50 % ou plus de la cholestérol oxydase sauvage.

**5.** Polynucléotide isolé codant la cholestérol oxydase mutante selon l'une quelconque des revendications 1 à 4.

**6.** Vecteur comprenant le polynucléotide selon la revendication 5.

**7.** Cellule hôte transformée avec un vecteur selon la revendication 6.

**8.** Procédé pour tester le cholestérol dans un échantillon, comprenant la mise en contact de l'échantillon avec la cholestérol oxydase selon l'une quelconque des revendications 1 à 4, et la mesure de la quantité de cholestérol oxydé par la cholestérol oxydase.

**9.** Procédé pour tester le cholestérol HDL comprenant la réaction du HDL dans un échantillon pour produire du cholestérol, la mise en contact du cholestérol avec la cholestérol oxydase selon l'une quelconque des revendications 1 à 4, et la mesure de la quantité de cholestérol oxydé.

**10.** Procédé pour tester le cholestérol LDL comprenant la réaction du LDL dans un échantillon pour produire du cholestérol, la mise en contact du cholestérol avec la cholestérol oxydase selon l'une quelconque des revendications 1 à 4, et la mesure de la quantité de cholestérol oxydé.

**11.** Dispositif pour tester le cholestérol, le cholestérol HDL et/ou le cholestérol LDL dans un échantillon comprenant la cholestérol oxydase selon l'une quelconque des revendications 1 à 4 et un médiateur d'électrons.

**12.** Kit pour tester le cholestérol, le cholestérol HDL et/ou le cholestérol LDL dans un échantillon comprenant la cholestérol oxydase selon l'une quelconque des revendications 1 à 4 et un médiateur d'électrons.

**13.** Électrode enzymatique ayant la cholestérol oxydase selon l'une quelconque des revendications 1 à 4 qui est immobilisée sur l'électrode.

**14.** Capteur enzymatique pour tester le cholestérol, le cholestérol HDL, le cholestérol LDL comprenant l'électrode enzymatique selon la revendication 13 comme électrode de travail.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2004113900 A
- US 5997817 A
- US 7008799 B
- US 6036919 A
- US 5334508 A

### Non-patent literature cited in the description

- **VRIELINK et al.** *FEBS J,* 2009, vol. 276 (23), 6826-6848
- **POLLEGIONI et al.** *FEBS J.,* 2009, vol. 276 (23), 6857-70
- **TOYAMA et al.** *Protein Engineering,* 2002, vol. 15 (6), 477-483
- **YAN SUN.** *Biotechnology Letters,* 2011, vol. 33 (10), 2049-2055
- **YUE et al.** *Biochemistry,* 1999, vol. 38 (14), 4277-4286
- **LU, G. ; MORIYAMA, E. N.** Vector NTI, a balanced all-in-one sequence analysis suite. *Brief Bioinform,* 2004, vol. 5, 378-88